# EUROPÄISCHE PATENTSCHRIFT

(11) **EP 0 918 743 B1**
(45) Veröffentlichungstag und Bekanntmachung des Hinweises auf die Patenterteilung: **03.04.2002**
(21) Anmeldenummer: 97925054.5
(22) Anmeldetag: 06.06.1997
(51) Int. Cl.: C07C 213/06, C07C 219/06, C07C 219/08

(54) **VERFAHREN ZUR HERSTELLUNG VON ESTERQUATS**
METHOD OF PREPARING QUATERNARY TRIALKANOLAMINE ESTER SALTS
PROCEDE DE PREPARATION DE COMPOSES QUATERNAIRES D'ESTERS

(30) Priorität: 12.06.1996 DE 19623325
(43) Veröffentlichungstag der Anmeldung: 02.06.1999
(73) Patentinhaber: Akzo Nobel N.V., 6824 BM Arnhem (NL)
(72) Erfinder: BERGFELD, Manfred, D-63906 Erlenbach (DE); AHRENS, Hartmut, D-63839 Kleinwallstadt (DE); CARSTENS, Axel, D-63739 Aschaffenburg (DE)
(74) Vertreter: Beetz, Tom
(86) Internationale Anmeldenummer: EP9702941
(87) Internationale Veröffentlichungsnummer: WO9747588

(56) Entgegenhaltungen:
- WO-A-91/01295
- DE-C- 4 409 322
- CHEMICAL ABSTRACTS, vol. 122, no. 15, 10.April 1995 Columbus, Ohio, US; abstract no. 186940x, YASUYUKI TAKAYANAGI ET AL.: "Manufacture of unsaturated quaternary ammonium salts" Seite 994; XP002041743 & JP 06 279 371 A (NITTO) 4.Oktober 1994

## Beschreibung

Die Erfindung betrifft ein Verfahren zur Herstellung von Esterquats durch direkte Veresterung von quaternären Ammoniumsalzen mit Fettsäuren.

Esterquats sind quaternäre Ammoniumverbindungen, die als Salze vorliegen und neben der quaternären Ammoniumfunktion eine Esterfunktion aufweisen.

Quaternäre Ammoniumsalze reagieren leicht unter Übertragung von Alkylgruppen oder unter Hofmann-Eliminierung. Oftmals werden beide Reaktionen nebeneinander beobachtet. Zur Hofmann-Eliminierung ist eine Base notwendig, zur Dealkylierung ein Nucleophil. Als Base bzw. als Nucleophil sind u.a. das Hydroxid-Ion und Halogenid-Ionen geeignet. Auch bei der Umsetzung von quatären Ammoniumverbindungen mit Carboxylaten geht eine O-Alkylierung des Carboxylats mit der entsprechenden Dealkylierung der Ammonium-Komponente einher. Je nach Reaktionspartner dominiert die Dealkylierung oder die Eliminierung (Hanhart and Ingold, J. Chem. Soc., 1927, 997. V. Meyer, M. Lecco, Liebigs Ann. 180, 184 (1876). W. Lossen, Liebigs Ann. 181, 377 (1876). J.A. Zoltewicz, L. W. Deady, Adv. Heterocycl. Chem. 22, 71 (1978). Lawson, Collie, J. Chem.Soc., 53, 624 (1888)).

Aus dem gleichen Grund sind quaternäre Ammoniumsalze als Phasentransfer-Katalysatoren nur bis Temperaturen von 100 - 150 °C stabil (D. Landini, A. Maia, A. Rampoldi, J. Org.Chem. 1986, 51, 3187-3191).

Auch Cholinchlorid zeigt diese für quatäre Ammoniumverbindungen typischen Eigenschaften. So zerfällt es beim Erhitzen in Dimethylaminoethanol und Methylchlorid (Ullmann's Encyclopedia of Industrial Chemistry, 5th edition, 1986, Vol. A7, 39) und setzt über eine Hofmann-Eliminierung Ethen frei (B.A. Kurchii, Fiziol. Biokhim. Kul't. Rast. 1991, 23 (1), 17-23. aus: Chemical Abstracts 1991, Vol. 114, 223451w).

In Russian Journal of Applied Chemistry, Vol. 67, No. 5, Part 2, 1994, 734-736 wird die Hofmann-Eliminierung von Cholinesterquats beschrieben.

Folglich ist bei der direkten Umsetzung von Cholinchlorid mit Fettsäuren sowohl die Hofmann-Eliminierung zu Trimethylamin und Ethen bzw. zum Vinylester der entsprechenden Fettsäure als auch eine Dealkylierung zu Dimethylaminoethanol bzw. zum Dimethylaminoethylester der entsprechenden Fettsäure und Methylchlorid bzw. Fettsäure-methylester zu erwarten. Zudem übt die quatäre Ammoniumgruppe einen -I-Effekt auf die Hydroxyfunktion aus, so daß diese für eine Veresterung desaktiviert ist (Methoden der organischen Chemie (Houben-Weyl) 1958, Stickstoff-Verbindungen II und III, 631).

Aus diesen Gründen wird die technische Synthese eines Cholinesters nicht über die Direktveresterung von Cholinchlorid mit der entsprechenden Carbonsäure vollzogen, sondern stattdessen wird in einer zweistufigen Synthese Dimethylaminoethanol zunächst mit der entsprechenden Säure umgesetzt und der Ester danach mit Methylchlorid oder Dimethylsulfat quaternisiert (Huadong Huagong Xueyuan Xuebao (1993), 19 (5), 594-9). Diese Quaternisierungsreagenzien sind stark toxisch und führen zusätzlich zu einer Methylierung des Lösungsmittels.

Eine einstufige Synthese ist in Izv. Vyssh. Ucheb. Zaved., Khim. Khim. Tekhnol. (1971), 14(9), 1369-73 beschrieben, wobei Cholinchlorid mit Fettsäurechloriden umgesetzt wird. Säurechloride müssen aber vorher aus den freien Säuren hergestellt werden, so daß der Rohstoffpreis wesentlich höher ist. Zudem sind sie korrosiv, feuchtigkeitsempfindlich und wegen ihrer hohen Reaktivität schwer handhabbar, so daß sie für die Technik nur unter erheblichem Aufwand einsetzbar sind. Folglich ist eine solche Cholinesterquat-Synthese unwirtschaftlich.

Auch die Umsetzung von Fettsäure(2-chlorethyl)ester mit Trimethylamin ist unwirtschaftlich, da vor der Quaternisierung noch der Ester hergestellt werden muß (Izv. Vyssh. Uchebn. Zaved., Khim. Khim. Tekhnol. (1977), 20(8), 1243-5).

Verbindungen dieser Art werden auch z. B. in der WO 91/01295 beschrieben. Entsprechend dem dort beschriebenen Verfahren führt man zunächst eine Veresterung einer Fettsäure mit einem Alkanolamin durch und quaternisiert den dabei erhaltenen Ester mit Substanzen wie Dimethylsulfat oder Methylchlorid in Lösungsmitteln wie Isopropanol. Von Nachteil bei derartigen Verfahren ist u.a., daß man dabei so toxische Substanzen wie Dimethylsulfat oder Methylchlorid verwendet. Ein weiterer Nachteil solcher Verfahren ist, daß das Lösungsmittel in der zweiten Stufe methyliert werden kann, z.B. Isopropanol zu Isopropylmethylether.

In Chemical Abstracts, Band 122 (1995), Abstract-Nr:186940x (JP,A,06,279,371) wird ein Verfahren zur Herstellung ungesättigter quaternärer Ammoniumsalze durch Veresterung von (Meth-)Acrylsäure mit quaternären Ammoniumsalzen in Gegenwart eines sauren Katalysators beschrieben. Die durch Polyphosphorsäure katalysierte Umsetzung von Acrylsäure mit Cholinchlorid ergibt eine Ausbeute von 65,9 % an Esterquat und einen auf Cholinchlorid bezogenen Umsatz von 66,8 % bei einem auf die Summe von Säure und Alkohol bezogenen Gewichtsanteil des Katalysators von 14,8 Gew.%.

Es besteht somit noch ein Bedürfnis nach einem verbesserten Verfahren zur Herstellung von Esterquats, bei dem die vorstehend aufgeführten Nachteile nicht auftreten.

Aufgabe der Erfindung ist es deshalb, ein Verfahren zur Herstellung von Esterquats zur Verfügung zu stellen, das ohne so stark toxische Substanzen wie Dimethylsulfat oder Methylchlorid arbeitet, das einstufig durchzuführen ist, von billigen Ausgangsstoffen ausgeht und bei dem Esterquats von hoher Reinheit entstehen, d.h. die keine oder nur in geringem Maße Nebenprodukte wie Esteramine oder Fettsäuremethylester und außer dem Hauptprodukt gegebenenfalls noch einen Teil freier Fettsäure enthalten.

Diese Aufgabe wird gelöst durch ein Verfahren zur Herstellung von Esterquats der Formel (I) 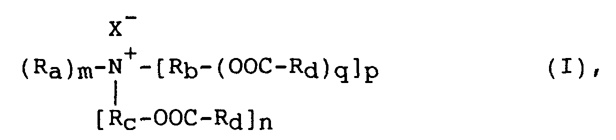 worin X⁻ ein Anion einer anorganischen oder organischen Säure, Rₐ eine C₁- bis C₄-Alkylgruppe, R_{b} und R_{c} unabhängig voneinander eine C₁- bis C₃-Alkylengruppe und R_{d} einen C₆- bis C₂₂-Rest einer gesättigten und/oder ungesättigten aliphatischen Carbonsäure bedeuten, m, n, p und q ganze Zahlen darstellen, m Werte von 1 bis 3, n Werte von 0 bis 3, p Werte von 0 bis 1 aufweist, m+n+p=4 ist und q den Wert 1 oder, wenn m=3, n=0 und p=1 ist, den Wert 2 annimmt, das dadurch gekennzeichnet ist, daß man eine quaternäre Verbindung der Formel (II) 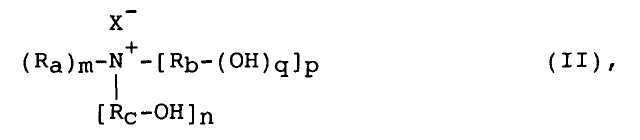 worin X⁻, Rₐ, R_{b}, R_{c}, m, n, p und q die oben angegebene Bedeutung haben, in Gegenwart einer Sauerstoffsäure des Phosphors und/oder eines ihrer Alkali- oder Erdalkalisalze als Katalysator mit einer gesättigten und/oder ungesättigten C₆- bis C₂₂-Carbonsäure einzeln oder im Gemisch unter Wasserentzug verestert und das dabei entstehende Reaktionsgemisch gegebenenfalls aufarbeitet.

Das während der Veresterung entstehende Wasser muß zur Steigerung des Umsatzgrades entfernt werden, z.B. im Vakuum oder durch Einsatz eines entsprechenden Wasserabscheiders.

Vorzugsweise wird im Vakuum von p ≤ 200 mbar gearbeitet.

Als Katalysator werden Sauerstoffsäuren des Phosphors, z.B. Diphosphorsäure, Metaphosphorsäure, Polyphosphorsäure, insbesondere Phosphorsäure, phosphorige Säure und hypophosphorige Säure oder deren Salze wie z.B. Na₃PO₄·10 H₂O, Mononatriumdihydrogenhypophosphit und Natriumhypophosphit-Monohydrat verwendet. Die Katalysatoren können entweder in Substanz oder als Lösungen eingesetzt werden, wobei wäßrige Lösungen bevorzugt werden.

Es ist vorteilhaft, die Veresterung mit einem Überschuß an Carbonsäure durchzuführen, der in weitesten Grenzen gewählt werden kann. Vorteilhafterweise werden pro Mol Cholinsalz mindestens 1,5, besonders bevorzugt 4 bis 20 Mol Carbonsäure eingesetzt.

Die Temperatur beträgt vorteilhafterweise mindestens 100 °C, wobei der Bereich 130 bis 220 °C bevorzugt und der Bereich 150 bis 170 °C besonders bevorzugt ist.

Die Veresterung läßt sich vorteilhaft in einem Reaktor durchführen, der einen Wasserabscheider aufweist.

Die Aufarbeitung des erhaltenen Reaktionsgemisches erfolgt in vorteilhafter Weise mit einem Dünnschichtverdampfer. Bei der Aufarbeitung wird zumindest ein Teil der überschüssigen Carbonsäure abgetrennt, wobei sich ein Dünnschichtverdampfer besonders eignet.

Als Ausgangssubstanz für die Veresterung werden quaternäre Verbindungen der Formel (II), z.B. Cholinchlorid verwendet. Cholinchlorid kann in einfacher Weise aus Trimethylamin, Ethylenoxid, Kohlensäure und Wasser hergestellt werden, wobei zunächst das Cholin-Hydrogencarbonat entsteht, das durch Ansäuern mit Salzsäure in das entsprechende Cholinchlorid übergeht.

Weitere verwendbare Cholinsalze werden u.a. in der französischen Patentschrift 736 107 beschrieben.

Zur Veresterung können übliche Carbonsäuren natürlicher Herkunft mit 6 - 22 Kohlenstoffatomen dienen, oder auch solche C₆ - C₂₂ Carbonsäuren, die auf synthetische Weise hergestellt werden. Es können auch Carbonsäuregemische besagter Säuren eingesetzt werden, u.a. auch solche, welche gesättigte Alkansäuren und Säuren mit ein oder mehreren Doppelbindungen enthalten.

Nach Abschluß der Veresterung wird das erhaltene Reaktionsgemisch vorzugsweise aufgearbeitet, d.h. u.a., überschüssige Carbonsäure kann abgetrennt werden. Dies geschieht vorzugsweise mittels Destillation, wobei eine Dünnschicht-Destillation besonders geeignet ist. Dadurch wird ein sehr reiner Rückstand von Esterquat erhalten, der je nach Destillationsbedingungen lediglich noch einen Teil der überschüssigen Carbonsäure und außerdem den Katalysator enthält.

Noch vorhandene Reste des Katalysators können im allgemeinen im Produkt verbleiben. Sie können aber auch in Neutralsalze überführt werden.

Es war besonders überraschend, daß es gemäß der Erfindung möglich war, durch direkte Veresterung von quaternären Verbindungen der Formel (II) Esterquats zu erhalten, denn die Hydroxyfunktion ist wegen der Ammoniumgruppe für eine Veresterung desaktiviert, und statt der Veresterung waren die oben erwähnten Zersetzungsreaktionen zu erwarten.

Weiterhin ist es nicht erforderlich, so umständliche Mehrstufenverfahren gemäß dem Stand der Technik durchzuführen, bei denen darüber hinaus giftige Substanzen eingesetzt werden müssen. Der Umsetzungsgrad der quaternären Verbindung der Formel (II) ist sehr hoch bei einer für Esterquats hohen Selektivität. Die Umsetzungsgrade, bezogen auf eingesetztes Cholinsalz, liegen im allgemeinen über 92 %. Der Anfall an Nebenprodukten ist gering. Restanteile können im allgemeinen vollständig beim Abdestillieren der Carbonsäure mit entfernt werden. Das Verfahren ist im technischen Maßstab gut handhabbar. Die mitverwendeten Katalysatoren können im allgemeinen im Endprodukt verbleiben.

Die bei der Veresterung eingesetzte überschüssige Carbonsäure kann leicht zurückgewonnen werden und für den Prozeß wieder eingesetzt werden.

Die Erfindung wird durch folgende Beispiele näher erläutert:

### Beispiel 1

In einem Dreihalskolben mit Wasserabscheider wurden 1163,9 g (5,62 mol) Kokossäure und 112 g (96proz.; 0,77 mol) Cholinchlorid unter Zugabe von 4 ml (50proz.; 0,04 mol) Hypophosphoriger Säure auf 160 °C erhitzt. Unter einem Vakuum von 6 mbar wurde das Reaktionsgemisch 4 Std. bei dieser Temperatur gerührt. Danach wurde ein großer Teil der nicht umgesetzten Fettsäure an einem Dünnschichtverdampfer abdestilliert. Es wurden 191,5 g eines hellen Feststoffes isoliert, der folgende Zusammensetzung aufweist:

| | |
|---|---|
| Cholinesterquat | 59.7 Gew.% |
| Cholinchlorid | 0.3 Gew.% |
| freie Fettsäure | 38.6 Gew.% |
| Hypophosphorige Säure | 1.3 Gew.% |

Der Umsetzungsgrad an Cholinchlorid berechnet sich somit zu 99.5 %.

### Beispiel 2

In einem Dreihalskolben mit Wasserabscheider wurden 32,03 g (0,16 mol) Laurinsäure mit 3,89 g (75proz.; 20,0 mmol) einer wäßrigen Cholinchlorid-Lösung unter Zugabe von 0,1 ml (85proz.; 1,5 mmol) Phosphorsäure auf 160 °C erhitzt. Unter einem Vakuum, das sich innerhalb von einer Stunde von 39 mbar auf 10 mbar aufbaute, wurde das Reaktionsgemisch 4 Std. bei dieser Temperatur gerührt. Dabei wurde ein Umsetzungsgrad bezogen auf Cholinchlorid von 96,4 % erzielt. Die Selektivität der Umsetzung zum Cholinesterquat beträgt 88,5 %, die Ausbeute an Cholinesterquat ist 85,3 %.

### Beispiel 3

In einem Dreihalskolben mit Wasserabscheider wurden 20,0 g (0,10 mol) Laurinsäure mit 2,9 g (96proz.; 20,0 mmol) Cholinchlorid unter Zugabe von 0,5 ml (50proz.; 4,8 mmol) einer wäßrigen Lösung von Hypophosphoriger Säure auf 160 °C erhitzt. Unter einem Vakuum, das sich innerhalb von einer Stunde von 39 mbar auf 10 mbar aufbaute, wurde das Reaktionsgemisch 4 Std. bei dieser Temperatur gerührt. Dabei wurde ein Umsetzungsgrad bezogen auf Cholinchlorid von 96,9 % erzielt. Die Selektivität der Umsetzung zum Cholinesterquat beträgt 96,5 %, die Ausbeute an Cholinesterquat ist 93,5 %.

### Beispiel 4

In einem Büchiautoklav mit Bodenablaß und Kondensator wurden 465,6 g (2,24 mol) Kokossäure mit 8 ml (50proz., 0,08 mol) Hypophosphoriger Säure versetzt und auf 150 °C erhitzt. Innerhalb von einer Stunde wurden bei 150 °C und einem Vakuum von 20 mbar mittels einer Schlauchpumpe insgesamt 57,2 g (75proz., 0,31 mol) einer wäßrigen Lösung von Cholinchlorid zudosiert. Danach wurde das Reaktionsgemisch noch 5 Std. unter einem Vakuum von 6 mbar bei dieser Temperatur gerührt. Schließlich wurde der Inhalt auf 100 °C abgekühlt, und der Autoklav wurde belüftet. Das Produkt wurde abgelassen. Dabei wurde ein Umsetzungsgrad bezogen auf Cholinchlorid von 99,3 % erzielt. Die Selektivität der Umsetzung zum Cholinesterquat beträgt 94,2 %, die Ausbeute an Cholinesterquat ist 93,5 %.

### Beispiel 5

In einem Dreihalskolben mit Wasserabscheider wurden 60,4 g (0,30 mol) Laurinsäure mit 2,8 g (96proz.; 19,3 mmol) Cholinchlorid unter Zugabe von 0,23 g (0,61 mmol) Trinatriumphosphat Dodecahydrat auf 160 °C erhitzt. Unter einem Vakuum von 150 mbar wurde das Reaktionsgemisch 4 Std. bei der eben genannten Temperatur gerührt. Dabei wurde ein Umsatz, bezogen auf Cholinchlorid, von 93 % erzielt. Die Selektivität der Umsetzung zum Cholinesterquat betrug 85,6 % bei einer Ausbeute an Cholinesterquat von 79,7 %.

### Beispiel 6

In einem Dreihalskolben mit Wasserabscheider wurden 60,3 g (0,30 mol) Laurinsäure mit 2,9 g (96proz.; 20,0 mmol) Cholinchlorid unter Zugabe von 0,13 g (0,61 mmol) Mononatriumdihydrogenhypophosphit auf 160°C erhitzt. Unter einem Vakuum von 150 mbar wurde das Reaktionsgemisch 4 Std. bei der eben genannten Temperatur gerührt. Dabei wurde ein Umsatz, bezogen auf Cholinchlorid, von 92,5 % erzielt. Die Selektivität der Umsetzung zum Cholinesterquat betrug 85,7 % bei einer Ausbeute an Cholinesterquat von 79,2 %.

### Beispiel 7

In einem Dreihalskolben mit Wasserabscheider wurden 20,2 g (100 mmol) Kortacid C70 mit 3,8 g (10 mmol) ACER 96S038 unter Zugabe von 0,5 g (5 mmol) Natriumhypophosphit-Monohydrat auf 150 °C erhitzt. Kortacid C70 ist ein Gemisch von Fettsäuren der Struktur R'-COOH, wobei R' C₈H₁₅, C₁₀H₂₁, C₁₂H₂₃, C₁₄H₂₅ und C₁₆H₂₇ bedeutet. ACER 96S038 ist ein Gemisch der Diole der Formeln (2a), (2b) und (2c) 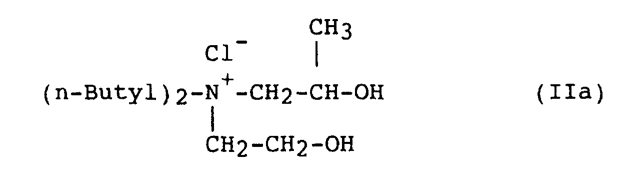 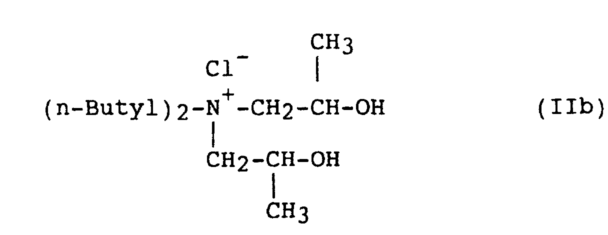 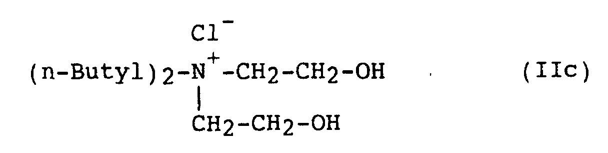 mit einem Molverhältnis der 2-Hydroxyethyl- zu den 2-Hydroxypropylgruppen von 9:1. Unter einem Vakuum, das sich innerhalb einer Stunde von 40 auf 10 mbar aufbaute, wurde das Reaktionsgemisch 6 Std. bei 150 °C gerührt. Laut ¹H-NMR-spektroskopischem Befund wurden 94 % der Hydroxygruppen verestert.

### Beispiel 8

Beispiel 7 wurde wiederholt mit dem Unterschied, daß 1,1 g (10 mmol) Natriumhypophosphit-Monohydrat eingesetzt wurden. Laut ¹H-NMR-spektroskopischem Befund wurden 96 % der Hydroxygruppen verestert.

### Beispiel 9

In einem Dreihalskolben mit Wasserabscheider wurden 20 g (0,10 mol) Laurinsäure mit 1,7 g (10 mmol) Dimethyl(bis-2-hydroxyethyl)ammoniumchlorid unter Zugabe von 0,5 ml (50proz.; 4,8 mmol) einer wässrigen Lösung von hypophosphoriger Säure auf 150 °C erhitzt. Unter diesem Vakuum, das sich innerhalb einer Stunde von 39 auf 10 mbar aufbaute, wurde das Reaktionsgemisch 6 Std. bei der eben genannten Temperatur gerührt. Dabei wurde ein Umsatz, bezogen auf das Dimethyl(bis-2-hydroxyethyl)ammoniumchlorid von 97,6 % erzielt. Die Selektivität der Umsetzung zum Diesterquatbetrug 70 %. Die Ausbeute an Diesterquat war 68,3 %.

### Beispiel 10

Beispiel 9 wurde wiederholt mit dem Unterschied, daß 1,0 ml (50proz.; 9,6 mmol) einer wässrigen Lösung von hypophosphoriger Säure verwendet. Dabei wurde ein Umsatz, bezogen auf das Dimethyl(bis-2-hydroxyethyl)ammoniumchlorid von 98,4 % erzielt. Die Selektivität der Umsetzung zum Diesterquatbetrug 79 %. Die Ausbeute an Diesterquat war 77,7 %.

### Beispiel 11

Beispiel 9 wurde wiederholt mit dem Unterschied, daß als Katalysator 0,5 g (5,0 mmol) Natriumhypophosphit-Monohydrat eingesetzt wurde. Dabei wurde ein Umsatz, bezogen auf das Dimethyl(bis-2-hydroxyethyl)ammoniumchlorid von 99,9 % erzielt. Die Selektivität der Umsetzung zum Diesterquat betrug 83,4 %. Die Ausbeute an Diesterquat war 83,3 %.

### Beispiel 12

Beispiel 11 wurde wiederholt mit dem Unterschied, daß als Katalysator 1,1 g (10,0 mmol) Natriumhypophosphit-Monohydrat eingesetzt wurde. Dabei wurde ein Umsatz, bezogen auf das Dimethyl(bis-2-hydroxyethyl)ammoniumchlorid von 98,5 % erzielt. Die Selektivität der Umsetzung zum Diesterquat betrug 89,7 %. Die Ausbeute an Diesterquat war 88,4 %.

### Beispiel 13

In einem Dreihalskolben mit Wasserabscheider wurden 20,0 g (0,10 mol) Laurinsäure mit 1,7 g (10,0 mmol) 2,3-Dihydroxypropyl-trimethylammoniumchlorid unter Zugabe von 1 ml (50proz.; 9,6 mmol) einer wässrigen Lösung von hypophosphoriger Säure auf 150 °C erhitzt. Unter einem Vakuum, das sich innerhalb einer Stunde von 39 auf 10 mbar aufbaute, wurde das Reaktionsgemisch 8 Std. bei der eben genannten Temperatur gerührt. Dabei wurde ein Umsatz, bezogen auf das 2,3-Dihydroxypropyl-trimethylammoniumchlorid von 96,5 % erzielt. Die Selektivität der Umsetzung zum Diesterquat betrug 71,5 %. Die Ausbeute an Diesterquat war 69,0 %.

### Beispiel 14

In einem Dreihalskolben mit Wasserabscheider wurden 20,0 g (0,10 mol) Laurinsäure mit 1,6 g (10 mmol) Tris(2-hydroxy-ethyl)methylammoniumchlorid unter Zugabe von 1,1 g (10 mmol) Natriumhypophosphit-Monohydrat auf 145 °C erhitzt. Unter einem Vakuum, das sich innerhalb einer Stunde von 40 auf 10 mbar aufbaute, wurde das Reaktionsgemisch 3 h bei der eben genannten Temperatur gerührt. Dabei wurde ein Umsatz, bezogen auf das Tris(2-hydroxyethyl)methylammoniumchlorid von 70,9 % erzielt. Die Selektivität der Umsetzung zum Triesterquat betrug 95,1 %. Die Ausbeute an Triesterquat betrug 67,4 %.

### Beispiel 15

Beispiel 14 wurde wiederholt mit dem Unterschied, daß das Reaktionsgemisch 4 Std. lang gerührt wurde. Die Ausbeute an Triesterquat betrug 75,0 %.

## Patentansprüche

1. Verfahren zur Herstellung von Esterquats der Formel (I) 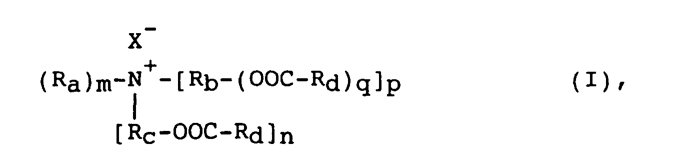 worin X⁻ ein Anion einer anorganischen oder organischen Säure, Rₐ eine C₁- bis C₄-Alkylgruppe, R_{b} und R_{c} unabhängig voneinander eine C₁- bis C₃-Alkylengruppe und R_{d} einen C₆-bis C₂₂-Rest einer gesättigten und/oder ungesättigten aliphatischen Carbonsäure bedeuten, m, n, p und q ganze Zahlen darstellen, m Werte von 1 bis 3, n Werte von 0 bis 3, p Werte von 0 bis 1 aufweist, m+n+p=4 ist und q den Wert 1 oder, wenn m=3, n=0 und p=1 ist, den Wert 2 annimmt, **dadurch gekennzeichnet, daß** man eine quaternäre Verbindung der Formel (II) 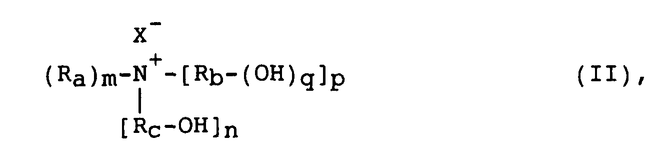 worin X⁻, Rₐ, R_{b}, R_{c}, m, n, p und q die oben angegebene Bedeutung haben, in Gegenwart einer Sauerstoffsäure des Phosphors und/oder eines ihrer Alkali- oder Erdalkalisalze als Katalysator mit einer gesättigten und/oder ungesättigten aliphatischen C₆- bis C₂₂-Carbonsäure einzeln oder im Gemisch unter Wasserentzug verestert und das dabei entstehende Reaktionsgemisch gegebenenfalls aufarbeitet.

2. Verfahren nach Anspruch 1, **dadurch gekennzeichnet, daß** man während der Veresterung Wasser laufend abführt.

3. Verfahren nach einem der Ansprüche 1 bis 2, **dadurch gekennzeichnet, daß** man das Wasser mittels Vakuum abführt.

4. Verfahren nach einem der Ansprüche 1 bis 3, **dadurch gekennzeichnet, daß** man die Veresterung bei einem Druck p ≤ 200 mbar durchführt.

5. Verfahren nach einem der Ansprüche 1 bis 4, **dadurch gekennzeichnet, daß** man als Sauerstoffsäure des Phosphors Phosphorsäure verwendet.

6. Verfahren nach einem der Ansprüche 1 bis 4, **dadurch gekennzeichnet, daß** man als Sauerstoffsäure des Phosphors phosphorige Säure verwendet.

7. Verfahren nach einem der Ansprüche 1 bis 4, **dadurch gekennzeichnet, daß** man als Sauerstoffsäure des Phosphors hypophosphorige Säure verwendet.

8. Verfahren nach einem der Ansprüche 1 bis 4, **dadurch gekennzeichnet, daß** man als Salz einer Sauerstoffsäure des Phosphors Na₃PO₄·10 H₂O, Mononatriumdihydrogenhypophosphit und Natriumhypophosphit-Monohydrat verwendet.

9. Verfahren nach einem der Ansprüche 1 bis 8, **dadurch gekennzeichnet, daß** man zur Veresterung der quaternären Verbindung der Formel (II) einen Überschuß an Carbonsäure verwendet.

10. Verfahren nach Anspruch 9, **dadurch gekennzeichnet, daß** man pro Mol an quaternärer Verbindung der Formel (II) mindestens 1,5, vorzugsweise 4 - 20 Mol Carbonsäure verwendet.

11. Verfahren nach einem der Ansprüche 1 bis 10, **dadurch gekennzeichnet, daß** man die Veresterung bei einer erhöhten Temperatur von mindestens 100 °C durchführt.

12. Verfahren nach Anspruch 11, **dadurch gekennzeichnet, daß** die Temperatur 130 bis 220 °C beträgt.

13. Verfahren nach Anspruch 12, **dadurch gekennzeichnet, daß** die Temperatur 150 bis 170 °C beträgt.

14. Verfahren nach einem der Ansprüche 1 bis 13, **dadurch gekennzeichnet, daß** man die Veresterung in einem Reaktionsgefäß mit Wasserabscheider durchführt.

15. Verfahren nach einem der Ansprüche 1 bis 14, **dadurch gekennzeichnet, daß** man vorhandene überschüssige Carbonsäure sowie Nebenprodukte ganz oder teilweise mittels eines Dünnschichtverdampfers vom erhaltenen Esterquat abtrennt.

## Claims

1. A process for the preparation of esterquats of formula (I) 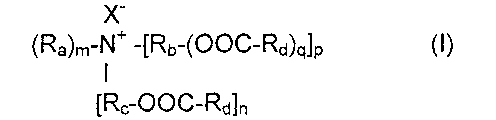 wherein X⁻ stands for an anion of an inorganic or organic acid, Rₐ stands for a C₁ - C₄ alkyl group, R_{b} and R_{c} may be the same or different and stand for a C₁ - C₃ alkylene group, and R_{d} stands for a C₆ - C₂₂ group of a saturated and/or unsaturated aliphatic carboxylic acid, m, n, p, and q represent integers, m has a value from 1 - 3, n has a value from 0 - 3, p has a value from 0 - 1, m+n+p=4, and q assumes the value 1 or, when m=3, n=0, and p=1, the value 2, which is **characterised in that** a quaternary compound of formula (II) 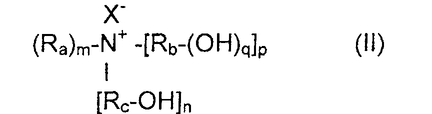 wherein X⁻, Rₐ, R_{b}, R_{c}, m, n, p, and q have the above-indicated meaning, is esterified in the presence of an oxyacid of phosphorus and/or one of the alkali or earth alkaline salts thereof as catalyst with a saturated and/or unsaturated C₆ - C₂₂ carboxylic acid individually or in a mixture with removal of water, and the thus formed reaction mixture is optionally worked up.

2. A process according to claim 1, **characterised in that** during the esterification water is continually discharged.

3. A process according to either of claims 1 - 2, **characterised in that** the water is discharged by means of a vacuum.

4. A process according to any one of claims 1 - 3, **characterised in that** the esterification is carried out at a pressure p ≤ 200 mbar.

5. A process according to any one of claims 1 - 4, **characterised in that** as oxyacid of phosphorus phosphoric acid is used.

6. A process according to any one of claims 1 - 4, **characterised in that** as oxyacid of phosphorus phosphorous acid is used.

7. A process according to any one of claims 1 - 4, **characterised in that** as oxyacid of phosphorus hypophosphorous acid is used.

8. A process according to any one of claims 1 - 4, **characterised in that** as salt of an oxyacid of phosphorus use is made of Na₃PO₄·10 H₂O, monosodium dihydrogen hypophosphite, and sodium hypophosphite-monohydrate.

9. A process according to any one of claims 1 - 8, **characterised in that** for the esterification of the quaternary compound of formula (II) use is made of an excess of carboxylic acid.

10. A process according to claim 9, **characterised in that** per mole of quaternary compound of formula (II) at least 1.5, preferably 4 - 20 moles of carboxylic acid are used.

11. A process according to any one of claims 1 - 10, **characterised in that** the esterification is carried out at an elevated temperature of at least 100°C.

12. A process according to claim 11, **characterised in that** the temperature is 130 to 220°C.

13. A process according to claim 12, **characterised in that** the temperature is 150 to 170°C.

14. A process according to any one of claims 1 to 13, **characterised in that** the esterification is carried out in a reaction vessel with a water trap.

15. A process according to any one of claims 1 to 14, **characterised in that** present excess carboxylic acid as well as side products are separated wholly or in part from the obtained esterquat by means of a thin-film evaporator.

## Revendications

1. Procédé de préparation d'esterquats de formule (I) : 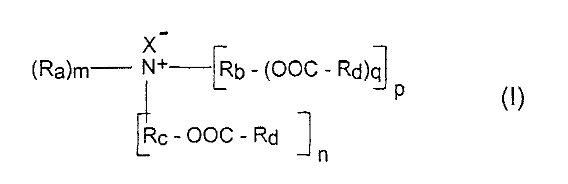 où X⁻ représente un anion d'un acide inorganique ou organique, Rₐ représente un radical alkyle en C₁ à C₄, R_{b} et R_{c} représentent indépendamment l'un de l'autre, un radical alkylène en C₁ à C₃, et R_{d} représente un reste en C₆ à C₂₂ d'un acide carboxylique aliphatique saturé et/ou insaturé, m, n, p et q représentent des nombres entiers, m présente des valeurs allant de 1 à 3, n des valeurs allant de 0 à 3, p des valeurs allant de 0 à 1, m+n+p=4 et q présente la valeur 1 ou, lorsque m=3, n=0 et p=1, accepte la valeur 2, qui est caractérisé en le que l'on estérifie un composé quaternaire de formule (II) : 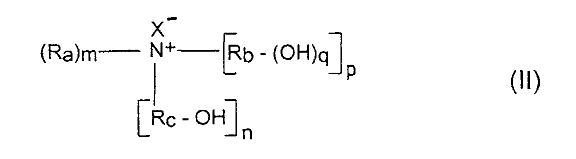 où X⁻, Rₐ, R_{b}, R_{c}, m, n, p et q ont la signification indiquée ci-dessus, en présence d'un acide oxygéné du phosphore et/ou d'un de ses sels alcalins ou alcalinoterreux comme catalyseur, avec un acide carboxylique saturé ou insaturé en C₆ à C₂₂ seul ou en mélange, avec élimination d'eau et en ce que l'on traite le cas échéant, le mélange réactionnel formé.

2. Procédé selon la revendication 1, **caractérisé en ce que** l'on élimine l'eau pendant l'estérification.

3. Procédé selon l'une quelconque des revendications 1 à 2, **caractérisé en ce que** l'on élimine l'eau par le vide.

4. Procédé selon l'une quelconque des revendications 1 à 3, **caractérisé en ce que** l'on réalise l'estérification à une pression p ≤ 200 mbar.

5. Procédé selon l'une quelconque des revendications 1 à 4, **caractérisé en ce que** l'on utilise comme acide oxygéné du phosphore, l'acide phosphorique.

6. Procédé selon l'une quelconque des revendications 1 à 4, **caractérisé en ce que** l'on utilise comme acide oxygéné du phosphore, l'acide phosphoreux.

7. Procédé selon l'une quelconque des revendications 1 à 4, **caractérisé en ce que** l'on utilise comme acide oxygéné du phosphore, l'acide hypophosphoreux.

8. Procédé selon l'une quelconque des revendications 1 à 4, **caractérisé en ce que** l'on utilise comme sel d'un acide oxygéné du phosphore, le Na₃PO₄.10H₂O, le dihydrogénophosphite de monosodium et l'hypophosphite de sodium monohydraté.

9. Procédé selon l'une quelconque des revendications 1 à 8, **caractérisé en ce que** l'on utilise pour l'estérification du composé quaternaire de formule (II), un excès d'acide carboxylique.

10. Procédé selon la revendication 9, **caractérisé en ce que** l'on utilise par mole du composé quaternaire de formule (II), au moins 1,5, de préférence 4 à 20 moles d'acide carboxylique.

11. Procédé selon l'une quelconque des revendications 1 à 10, **caractérisé en ce que** l'on réalise l'estérification à une température élevée, d'au moins 100°C.

12. Procédé selon la revendication 11, **caractérisé en ce que** la température se situe dans l'intervalle allant de 130 à 220°C.

13. Procédé selon la revendication 12, **caractérisé en ce que** la température se situe dans l'intervalle allant de 150 à 170°C.

14. Procédé selon l'une quelconque des revendications 1 à 13, **caractérisé en ce que** l'on réalise l'estérification dans un récipient de réaction avec séparateur d'eau.

15. Procédé selon l'une quelconque des revendications 1 à 14, **caractérisé en ce que** l'on sépare l'acide carboxylique en excès présent, ainsi que les produits secondaires, de l'esterquat obtenu, complètement ou partiellement à l'aide d'un évaporateur en couche mince.
